# EUROPEAN PATENT APPLICATION

(11) **EP 1 742 044 A2**
(43) Date of publication of application: **10.01.2007**
(21) Application number: 06014046.4
(22) Date of filing: 06.07.2006
(51) Int. Cl.: G01N 27/414

(54) **Semiconductor gas sensor and method for manufacturing the same**

(30) Priority: 08.07.2005 JP 2005199657
(71) Applicant: HITACHI, LTD., Chiyoda-ku Tokyo 100-8280 (JP)
(72) Inventor: Goto, Yasushi c/o Hitachi Ltd. Int. Prop. Group, Tokyo 100-8220 (JP); Mine, Toshiyuki c/o Hitachi Ltd. Int. Prop. Group, Tokyo 100-8220 (JP); Yokosawa, Koichi c/o Hitachi Ltd. Int. Prop. Group, Tokyo 100-8220 (JP)
(74) Representative: Beetz & Partner

(57) **Abstract**

The present invention provides a semiconductor gas sensor and a manufacturing method for the same, enabling suppression of threshold voltage fluctuation without giving any damage to a gate insulating film when a transistor structure (314) is formed at first in a field effect transistor type of gas sensor and then an electrode (425) with a material responsive to a gas to be detected is formed.

The gate insulating film is a film stack including at least an SiO₂ film (104;402) and an SRN (Si-rich nitride) film (107;406). The SRN film (107;406) functions as a etching stopper film when the gate insulating film (104;402) is exposed by etching of an inter-layer insulating film (108;412). Pressure resistance of the gate insulating film is preserved with SiO₂. An electric charge in the SRN film can be removed with a lower voltage as compared to that required for removing an electric charge in the Si₃N₄ film, which enables suppression of threshold voltage fluctuation in gas sensor transistors.

## Description

### BACKGROUND OF THE INVENTION

### FIELD OF THE INVENTION

The present invention relates to a device structure of a semiconductor gas sensor of field-effect transistor type and a method of manufacturing the semiconductor gas sensor.

### DESCRIPTION OF THE RELATED ART

It is known that a field-effect transistor having a gate electrode made of a material sensitive to a gas functions as a gas sensor. Already in 1975, I. Lundström et al. reported that a MOS (Metal-Oxide-Semiconductor) transistor having a gate electrode made of palladium has the responsiveness to hydrogen (Refer to I. Lunström et al., "A Hydrogen-sensitive MOS field-effect transistor", Applied Physics Letter, 1975, Vol. 26, No. 2, p. 55). In this report, the hydrogen sensor has a gate insulating film made of silicon oxide and having a thickness of 10 nm thereon and palladium is deposited with a thickness of 10 nm to form a MOS structure. When hydrogen is dissociated by palladium functioning as a gate electrode into the atomic state, diffused in the palladium, and is absorbed onto a surface of the insulating film, conductivity of the MOS transistor channel changes due to polarization of the atomic hydrogen, which in turn cause a change of a current between the source electrode and the gate electrode. Furthermore, Japanese Patent Laid-Open No. 62-237347 discloses a method enabling detection of even a fine quantity of reductive gas in the atmospheric air with high responsiveness and sensitivity by combining a gate electrode with a solid state ion conductor. WO 00/075649 discloses that, in addition to the silicon oxide film SiO₂, also aluminum oxide Al₂O₃ and tantalum oxide Ta₂O₅ may be used as a gate insulating film. In any of the cases described above, when an electrode on the gate insulating film is made of a responsive material, the responsiveness to hydrogen or other gases is provided.

Furthermore, a method is disclosed in which, when the gas sensor as described above is manufactured, an FET (Field-Effect Transistor) structure is previously formed and then a responsive material is added to the gate insulating film (Refer to N. Miura et al., "Sensing characteristics of ISFET-based hydrogen sensor using proton-conductive thick film", Sensors and Actuators, 1995, B24-25, p. 499). In this case, a material for a gate electrode in a MOS transistor can be changed according to a gas to be detected, and sensors responsive and sensitive to various types of oxidizing and reductive gases can be manufactured.

### SUMMARY OF THE INVENTION

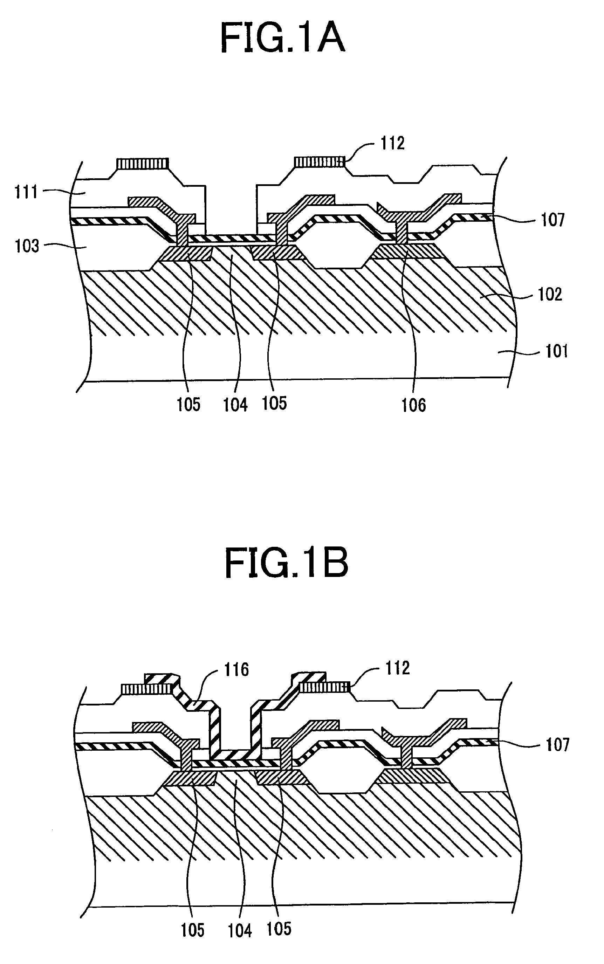

As shown in FIG. 1, when a transistor structure is formed at first (Refer to FIG. 1A) and then a gas-sensitive gate electrode is formed according to a gas to be detected (Refer to FIG. 1B), two functions are required to the gate insulating film. One of the two required functions is to function as a gate insulating film for a transistor, and another one is to function as an etching stopper when an inter-layer insulating film, which is an upper layer of the gate insulating film, or a passivation film is removed by etching process. Silicon oxide is generally used for the inter-layer insulating film, and it is desirable that a material having the etching selectivity to silicon oxide be used to form the gate insulating film or to form a film on the gate insulating film. In a process of manufacturing transistors, a gate insulating film for each transistor is formed in the initial stage of the process, and therefore generally a silicon-based insulating film is desirable. In the gate insulating film as described above, generally silicon nitride Si₃N₄ is generally used as an etching stopper having the selectivity ratio to the inter-layer insulating film, namely silicon oxide. This film contains Si and N, and does not contain any contaminant negatively affecting the device performance.

The gas sensor shown in FIG. 1B can be manufactured by forming a film stack of SiO₂ and Si₃N₄ as the gate insulating film and depositing on the film stack a material having sensitivity to a gas such as, for instance, palladium having the sensitivity to hydrogen. However, this structure is referred to as the MNOS (Metal-Mitride-Oxide-Semiconductor) structure, and is the same as that known as a device for accumulating charge in the Si₃N₄ film. Also the experiments conducted by the inventors confirmed that the threshold fluctuation in product transistors is larger, namely several volts, in the sensor (FIG. 1B) made by sputtering palladium to the structure shown in FIG. 1A. It can be considered that this phenomenon occurs due to injection of an electric charge to Si₃N₄ during removal of the inter-layer insulating film on the gate insulating film or during formation of the palladium film. It is possible to remove the electric charge by applying a voltage to a section between the gate electrode and the Si substrate, but for removal of the electric charge in the Si₃N₄ film, it is necessary to apply a voltage of 5 MV/cm or more, and stability of the sensor becomes lower because the gate insulating film is damaged.

An object of the present invention is to suppress fluctuation of device performance even when a gate electrode containing a material sensitivity to a gas is formed, after a transistor structure is formed at first, on the gate insulating film.

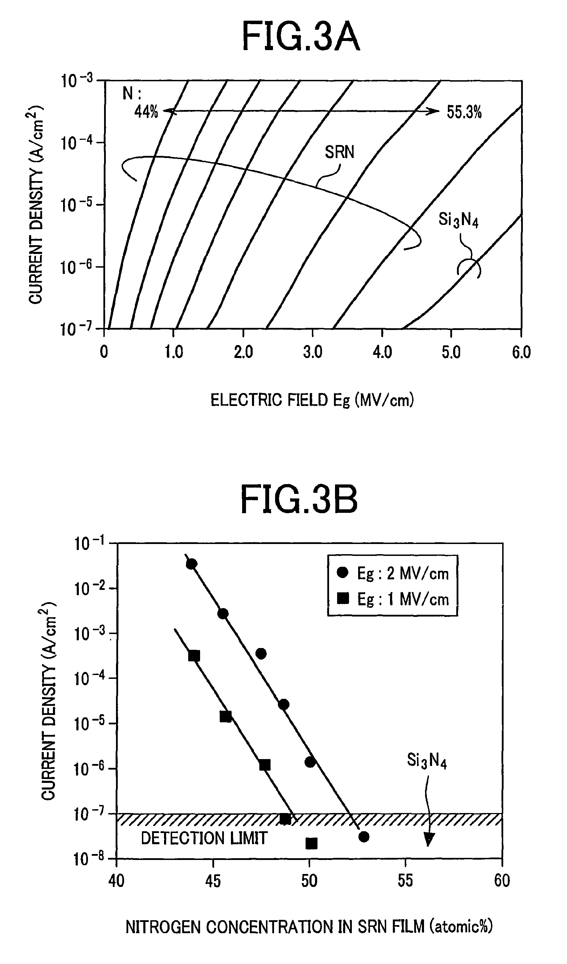

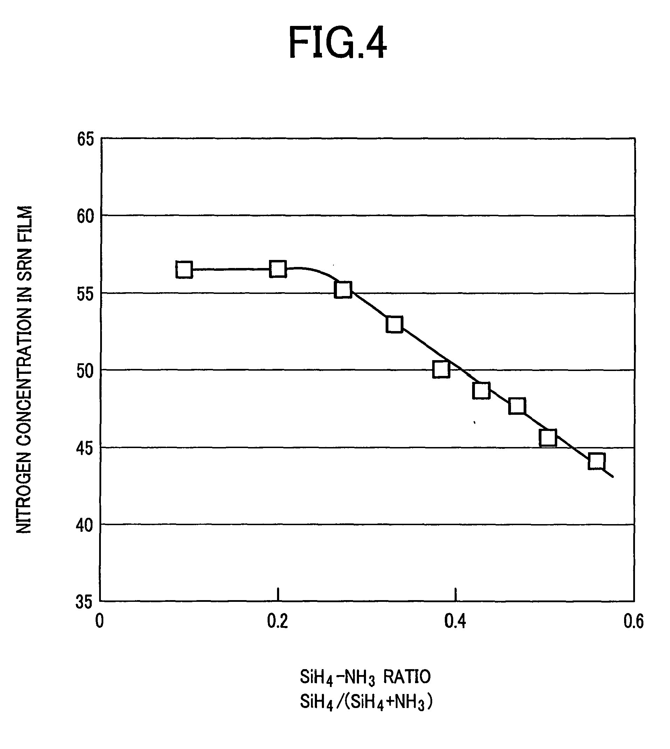

When a transistor structure is formed at first and then a gate electrode with a gas-sensitive material is formed, it is necessary to reduce an electric charge in the gate insulating film. For removal of the inter-layer insulating film on the gate insulating film, an etching stopper film is required, and the Si₃N₄ film is suitable as described above. The inventors focused attention to a ratio between Si and N in the Si₃N₄ film, and confirmed the fact that, when a content of Si in silicon nitride increases, a defective level occurs in the film and an electric charge in a silicon nitride film can be discharged by applying a low voltage. To differentiate the silicon nitride film with a higher Si content from the Si₃N₄ film, a silicon nitride film in which a composition ratio of silicon versus nitrogen is higher than 3/4 is referred to as SRN (Silicon-Rich Nitride) film hereinafter. By using this SRN film in place of a silicon nitride (Si₃N₄) film as a gate insulating film for a gas sensor, negative influence of electrification of the gate insulating film can be reduced. The insulating property of the SRN film and control over the Si:N ratio are described below with reference to FIG. 3 and FIG. 4.

The Si:N ratio in the SRN film can be controlled by adjusting a flow rate ratio between feed gases when a film is formed by making use of chemical vapor deposition. The Si:N ratio can also be controlled by reactive sputtering, but the SRN film also functions as an etching stopper when an inter-layer insulating film is worked, and the selectivity ratio for inter-layer insulating film working is better in the SRN film formed by means of the chemical vapor deposition as compared to that by other methods. SiH₄, NH₃, and N₂ were used for feed gasses for chemical vapor deposition. A flow rate of NH₃ was kept constant, while a flow rate of SiH₄ was changed. A flow rate of N₂ was adjusted so that the overall flow rate was constant. The intensity of N in the SRN film was measured with a fluorescent X-ray analysis. FIG. 4 illustrates a relation between a gas ratio of SiH₄ versus NH₃ in the feed gas and an N content in the SRN film. When the gas ratio R (a flow rate of SiH₄/a flow rate of SiH₄ + a flow rate of NH₃) is set at 0.2 or more, the N content becomes lower than the N content of about 57 atomic % in the Si₃N₄ film. Our experiment demonstrated that the N content is lowered to around 45 atomic %.

The electrical characteristics corresponding to each of the N contents checked in the experiment was accessed. An SRN film was deposited on a silicon substrate and Au was deposited as an upper electrode. In this structure, the current-voltage property was measured. FIG. 3A illustrates a relation between a field intensity between the upper and lower electrodes on the SRN film and a density of a current flowing in the SRN film. It is understood from a result of this experiment that a current flows more easily in an SRN film having the Si/N ratio (composition ratio) in the Si₃N₄ film higher than 3/4. As the N content becomes lower, namely when the Si concentration in the SRN film becomes higher, the tendency like those shown by the lines in the right side of the figure is obtained. From this fact, it can be considered that, when the Si:N ratio in Si₃N₄ is higher even a little, namely when the Si/N ratio is set higher than 3/4, a current flows more easily, which is conceivably effective for reduction of the threshold fluctuation. FIG. 3B shows a relation, when the electric field applied to an SRN film is 1 MV/cm or 2 MV/cm, between a density of a current flowing in the SRN film and a content (atomic %) of N in the SRN film. When the field intensity Eg is 2 MV/cm, the current intensity of 1 µA/cm² flows for the N content of about 50 atomic %, and in this case, the electric charge can be removed relatively easily. Especially, when the N contents is 50 atomic % or below, the effect is remarkable. When the field intensity Eg is 1 MV/cm, a current can be made to flow with the intensity of 1 µA/cm² for the N content of about 48 atomic % or below. When a higher current can be made to flow by applying a lower voltage, an electric charge in the SRN film can be removed more easily, which is effective in suppression of damage to the device. As shown above, an SRN film is stacked with an SiO₂ film to form a gate insulating film. The SRN film is required to function as an etching stopper when a silicon oxide inter-layer insulating film as an upper layer in the SRN film is worked. The Si content in the SRN film is higher than that in the Si₃N₄ film, and therefore the selectivity ratio for work of a silicon oxide film can be made higher. Because of this feature, a thickness of the SRN film may be smaller as compared to that of the Si₃N₄ film. The thickness of the SRN film of several tens nanometers is sufficient for the SRN film to function as an etching stopper film. Since the insulating property of the SRN film is low, the electric insulation capability is required to be protected with silicon oxide SiO₂. The thickness of the SiO₂ film for this purpose is 5 nanometers or more.

As described above, by forming a film stack of SRN and SiO₂ films as a gate insulating film as described above, it is possible to make the SRN film function as an etching stopper when an inter-layer insulating film is worked, to realize a gate insulating film having excellent electric insulation capability, and also to reduce threshold voltage fluctuation in sensor MOS transistors. Because of the features as described above, also fluctuation of the sensor property itself can be suppressed, which enables stable supply of sensors with high reliability.

### BRIEF DESCRIPTION OF THE DRAWINGS

FIG. 1A and FIG. 1B are cross-sectional views illustrating a gas sensor according to the present invention;

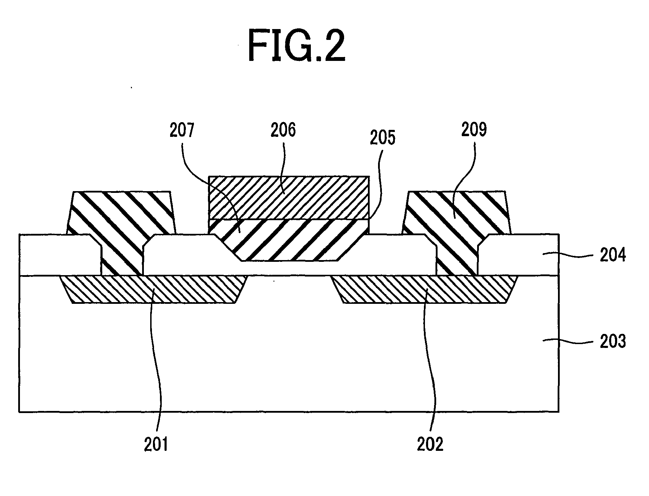

FIG. 2 is a cross-sectional view illustrating a gas sensor based on the related art;
FIG. 3A and FIG. 3B are views each illustrating the characteristics of an SRN film according to the present invention;
FIG. 4 is a view illustrating control over a Si/N ratio in the SRN film according to the present invention;
FIG. 5A to FIG. 5C are cross-sectional views each illustrating a portion of a process of manufacturing a gas sensor in a first embodiment of the present invention;
FIG. 6A to FIG. 6C are cross-sectional views each illustrating a portion of the process of manufacturing process of the gas sensor in the first embodiment of the present invention;
FIG. 7A and FIG. 7B are cross-sectional views each illustrating a portion of the process of manufacturing process of the gas sensor in the first embodiment of the present invention;
FIG. 8A to FIG. 8C are plan views each illustrating the process of manufacturing process of the gas sensor in the first embodiment of the present invention;
FIG. 9A to FIG. 9C are plan views illustrating a portion of the process of manufacturing process of the gas sensor in the first embodiment of the present invention;
FIG. 10 is a view illustrating the electrical characteristics of the gas sensor in the first embodiment of the present invention;
FIG. 11 is a view illustrating the dependence of a the gas sensor in the first embodiment of the present invention on a hydrogen concentration;
FIG. 12 is a cross-sectional view illustrating a portion of the process of manufacturing the gas sensor in the first embodiment of the present invention;
FIG. 13A to FIG. 13C are cross-sectional views each illustrating a portion of a process of manufacturing the gas sensor in a second embodiment of the present invention;
FIG. 14A to FIG. 14C are cross-sectional views each illustrating a portion of the process of manufacturing the gas sensor in the second embodiment of the present invention;
FIG. 15A and FIG. 15B are cross-sectional views illustrating a portion of the process of manufacturing the gas sensor in the second embodiment of the present invention;
FIG. 16A and FIG. 16B are cross-sectional views each illustrating a portion of the process of manufacturing the gas sensor in the second embodiment of the present invention; and
FIG. 17 is a cross sectional view illustrating a portion of the process of manufacturing the gas sensor in the second embodiment of the present invention.

### DESCRIPTION OF THE PREFERRED EMBODIMENTS

Preferred embodiments of the present invention are described in detail below.

### [First Embodiment]

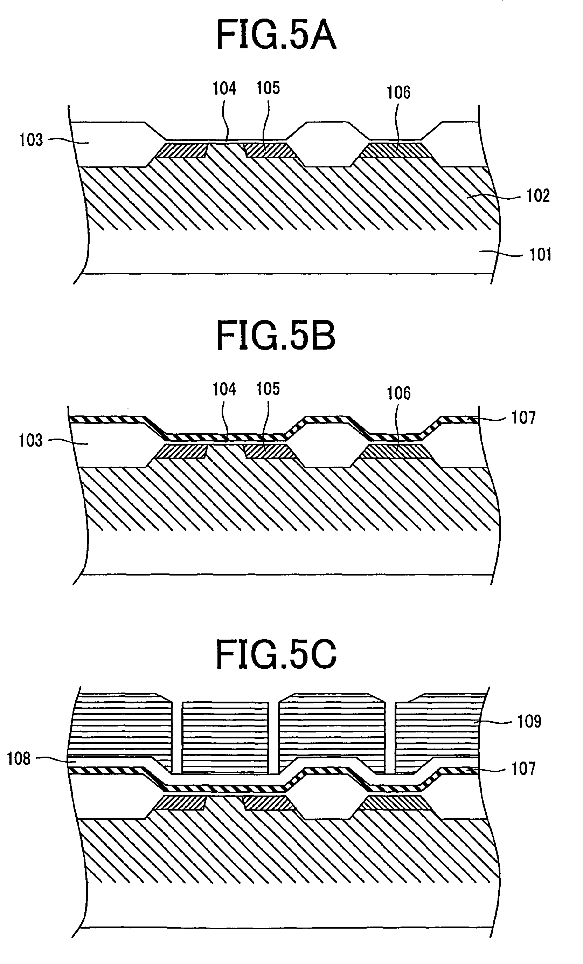

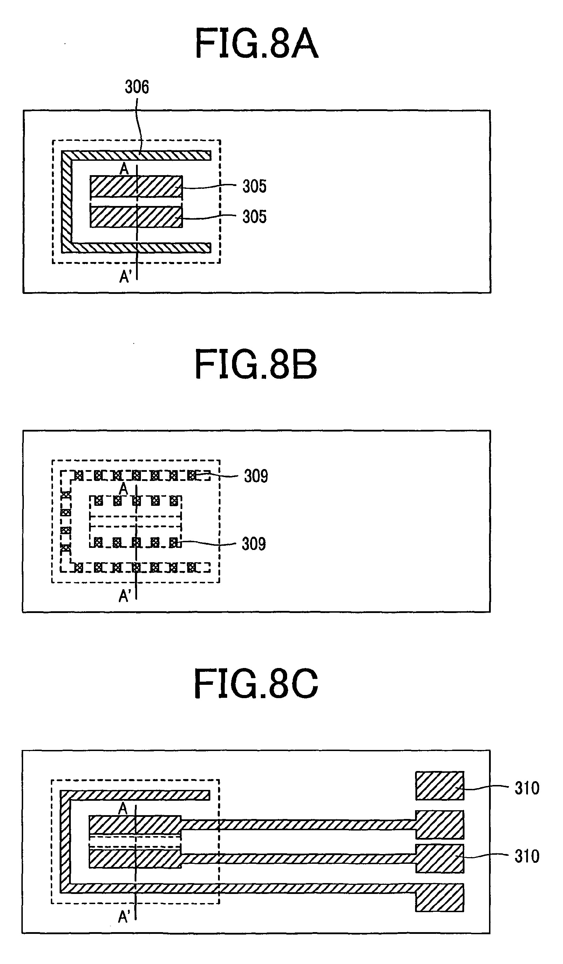

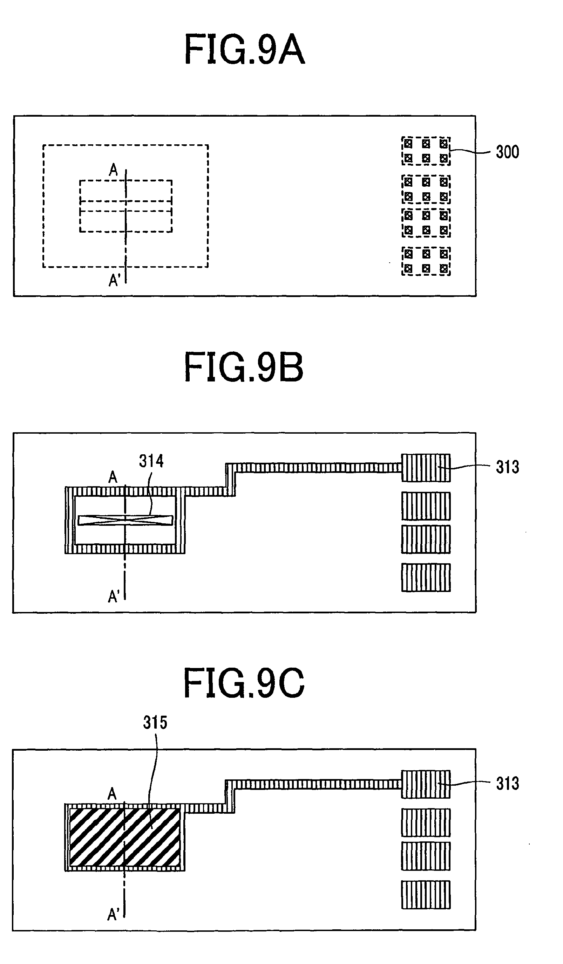

A method of producing a hydrogen sensor using palladium as a gas-responsive material is described below, as a first embodiment of the present invention, with reference to cross-sectional views shown in FIGS. 5A-5C to FIGS. 7A-7C as well as to plan views shown in FIGS. 8A-8C and FIGS. 9A-9C. FIG. 5A illustrates the situation in which a p-type well 102 is formed on a silicon substrate 101, an isolation 103 is formed, an SiO₂ film 104 as a portion of a gate insulating film is oxidized, and a source/drain drain diffusion layer 105 of a MOS transistor, and a p+ diffusion layer 106 for providing contacts with the p-type well 102 afterward are formed thereon. Although not shown in the figure, an n-channel is formed under the SiO₂ film 104 of the gate insulating film to control a threshold voltage of the transistor. This figure corresponds to a cross section of taken along the line the A-A' in the plan shown in FIG. 8A. A source/drain diffusion layer 305 is arranged in the p-type well indicated by a dotted line, and a p+ diffusion layer 306 is arranged so as to surround an outer circumference of the source/drain diffusion layer 305. A potential gradient distribution in the p-type well can be controlled by arranging the p+ diffusion layer 306 so as to surround the outer circumference as described above. Then an SRN film 107 as a portion of the gate insulating film is stacked as shown in FIG. 5B. Furthermore, an SiO₂ film as an inter-layer insulating film 108 is stacked on the SRN film 107 by means of the CVD, and a resist pattern 109 for connecting wiring to each of the diffusion layers is exposed as shown in FIG. 5C. These resist patterns 309 are arranged so as to be opened at a plurality of positions for the sources/drain diffusion layer and the p+ diffusion layer as shown in FIG. 8B.

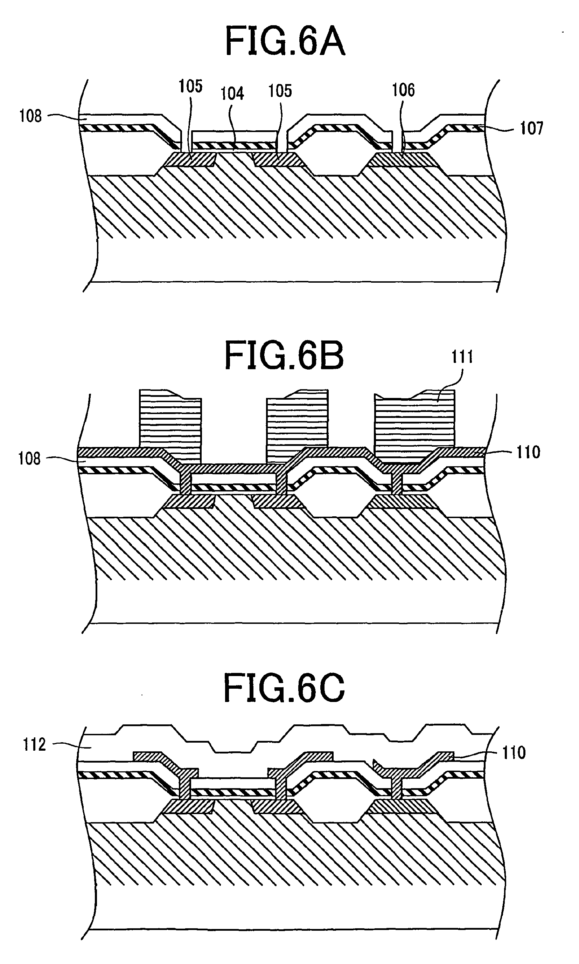

FIG. 6A illustrates the situation in which the inter-layer insulating film 108, the SRN film 107 and the SiO₂ film 104 are removed by dry etching using the resist patterns as a mask and then the resist is removed. After cleaning, Ti, TiN, and W films are successively formed into a first wiring layer 110 and a resist pattern 111 associated with each wiring is formed (FIG. 6B). Layout of the pattern is shown in FIG. 8C. A Pad electrode is provided on the place where the rectangular portions at the right end of the resist pattern 310 are provided. FIG. 6C illustrates the situation in which the first wiring layer 110 is subjected to dry etching by using the resist pattern as a mask, the resist is removed, cleaning is performed, and then a second insulating film 112 is stacked. A plasma nitride film for passivation is used as a second insulating film 112. Although not shown in the cross-sectional views, photolithography, etching, and cleaning processes are performed so as to form a through-hole film for providing the Pad electrode out from the first wiring layer 110. A resist pattern 300 for these through-holes is shown in FIG. 9A. A plurality of hole patterns are arranged only at the Pad electrode portion of the pattern 310 shown in FIG. 8C.

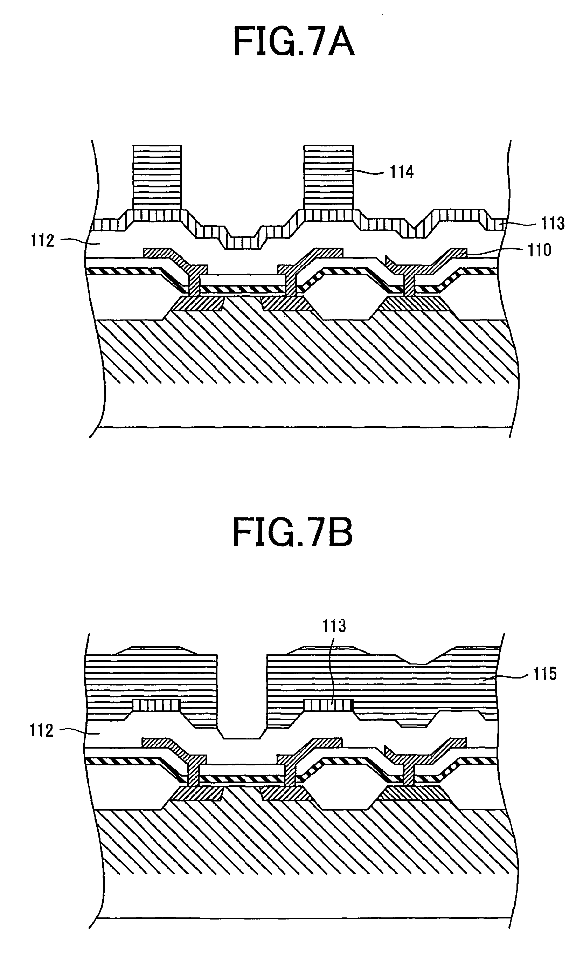

FIG. 7A illustrates the situation in which an Al film 113 functioning as the Pad electrode of a sensor is stacked, and a resist pattern 114 associated with the electrode structure is formed. FIG. 9B illustrates a resist pattern 313 for an electrode formed of the Al film. The top electrode is arranged up to a MOS transistor area 314 and is arranged so as to surround the transistor. FIG. 7B illustrates the situation in which the Al film 113 is worked by using the resist pattern 114 as a mask, the resist is removed, cleaning is performed, and a resist pattern 115 associated with the transistor area 314 in the FIG. 9B is formed.

A second insulating film 112 and an inter-layer insulating film 108 are removed by dry etching using the resist pattern 115 as a mask. The SRN film 107 functions as an etching stopper film in this step. Furthermore, the resist is removed; cleaning is performed; and palladium as a gas-responsive material is deposited as an electrode film 116 to form the gas sensor shown in FIG. 1. Palladium 315 is deposited to form a film so that the palladium film is overlaid on the Al film arranged on the outer circumference of the transistor area 314 as shown in FIG. 9C so as to be electrically connected to the Pad electrode at the right end. The palladium is deposited by sputtering deposition through with a stencil mask. Although a film with a high resistance value can be obtained when deposited at the room temperature during the film deposition, the resistance value of the film drops when the substrate is heated at the time of film deposition, and therefore a film having a good quality can be formed. The film quality is improved at a temperature of 150°C or higher in the embodiment.

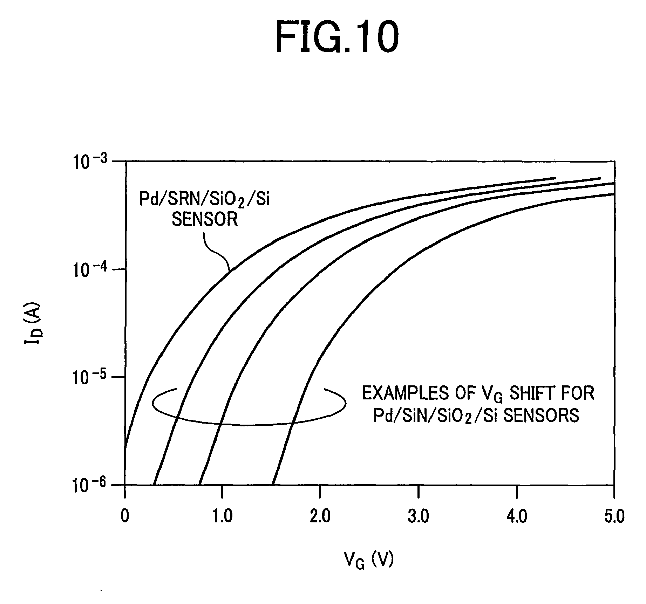

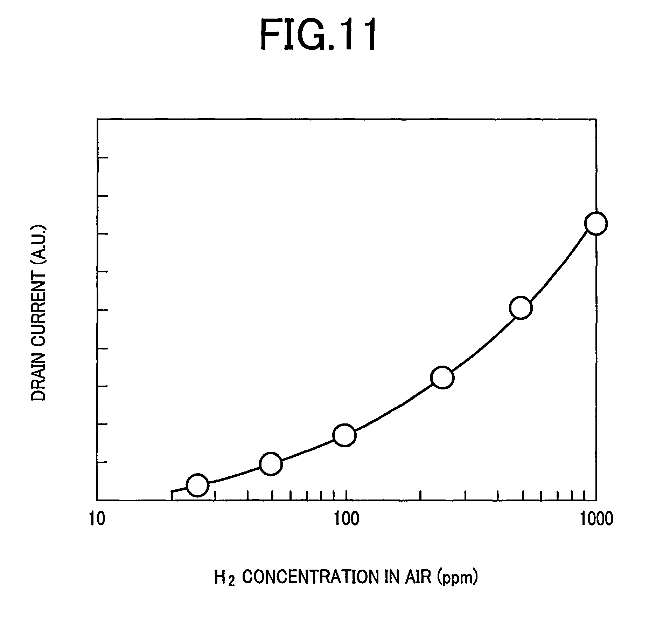

FIG. 10 shows the relationship between a gate voltage VG and a drain current ID in the transistor manufactured by the method according to the present invention. The source voltage is 0 V and the drain voltage is 1.5 V. In this embodiment, an n-type layer is formed in a channel area with a gate length of 30 µm and a gate width of 500 µm, and a drain current of several tens µA flows at a gate voltage of 0 V. When palladium 315 is deposited on a transistor with the ordinary Si₃N₄ film with a thickness of 50 nanometers deposited on a SiO₂ film with a thickness of 15 nanometers by sputtering deposition with a stencil mask in the process shown in FIG. 9C, a fluctuation of about 1.5 V was observed in the VG-ID property. The fluctuation is not reduced even when a voltage of about 5 V is applied between the gate electrode and the substrate. When a voltage of about 5 V is applied between the gate electrode and the substrate in the transistor with an SRN film with a thickness of 35 nanometers having the N content of about 48 atomic % in place of the Si₃N₄ film deposited on SiO₂ with a thickness of 15 nanometers, substantially no fluctuation was observed in the VG-ID property. As described above, when a palladium electrode is formed, the responsiveness to hydrogen can be obtained, and therefore the sensor functioning as a hydrogen gas sensor is obtained. FIG. 11 shows dependence of the drain current of the hydrogen sensor manufactured by the method on a hydrogen concentration. In this embodiment, a temperature of the sensor is set at 100°C in order to prevent influence by the humidity and changes in the drain current were investigated by blowing air containing hydrogen at various concentrations to the sensor portion. A gate voltage and a source voltage are set at 0 V, and a drain voltage is set at 1.5 V. As a result, it was confirmed in this experiment that the sensor shows the sensitivity to hydrogen even when a concentration of hydrogen is about 25 ppm.

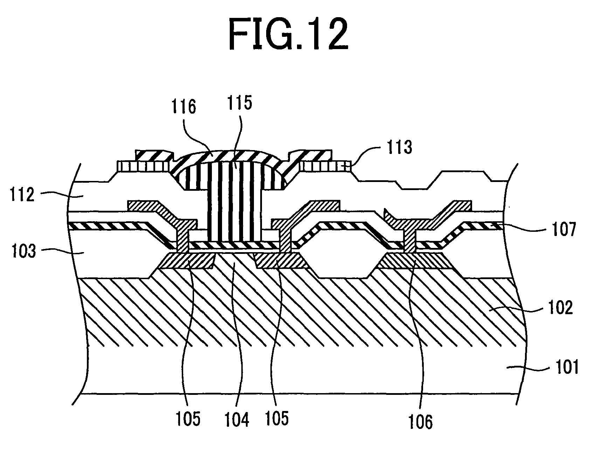

The description has been made above with reference to the hydrogen sensor using palladium as a gas-responsive material. Unlike the case in which the second insulating film 112 and the inter-layer insulating film 108 are worked by using the resist pattern 115 as a mask and using the SRN film 107 as a stopper, the resist is removed, and cleaning is performed and palladium is directly deposited as shown in FIG. 7B, a proton conductor 117 having the hydrogen selectivity is formed on the gate area, and then the palladium electrode film 116 can be formed so as to be connected to the Al film 113 on the outer circumferential portion while covering the proton conductor as shown in FIG. 12.

The hydrogen sensor using palladium or using palladium and a proton conductor as a responsive material is described in this embodiment. Also, hydrogen can be detected by using platinum in place of palladium. Furthermore, the sensor can be used for detecting a gas containing hydrogen such as methane.

Furthermore, the sensor can respond even to oxygen and CO by using a film stack of platinum and zirconium oxide as a responsive material. In other words, a gas sensor suitable for various gases can be manufactured by forming a transistor structure at first and then forming a gate electrode with a material responsive to a gas to be detected.

### [Second Embodiment]

A method for configuring a transistor circuit for detection or the like on the same substrate surface as that of the sensor is described below as a second embodiment of the present invention.

Since a current flows in the SRN film more or less, when the size of the transistor constituting the circuit is small, namely 0.35 micrometers or below, the SRN film in the circuit area is desirably removed.

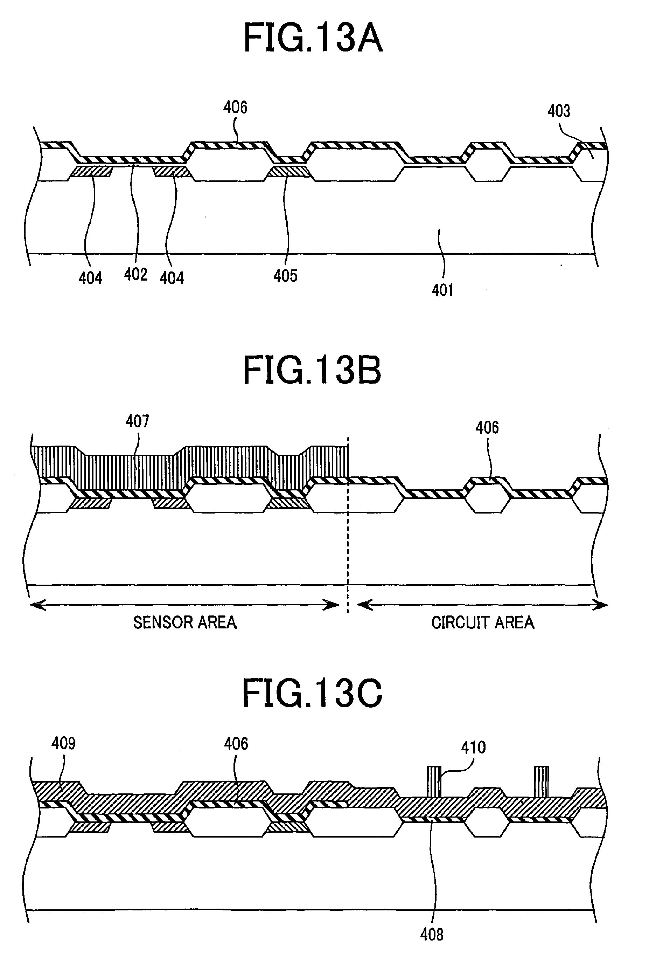

FIG. 13A illustrates the situation in which a p-type well is formed on a silicon substrate 401; an isolation 403 is formed; an SiO₂ film 402 as a portion of the gate insulating film is oxidized; and an SRN film 406 is deposited on a surface on which source/drain diffusion layer 404 of the MOS transistor as a sensor and a p+ diffusion layer 405 for taking contacts with the well afterward are formed. Because a current flows in the SRN film 106 more or less as described above, it is necessary to remove a silicon-rich nitride film of a circuit area, and the operation is described below. A resist pattern 407 is formed so that only the sensor area is covered with the resist pattern as shown in FIG. 13B. The SRN film 406 is dry-etched by using this resist pattern 407 as a mask, the resist is removed; cleaning is performed, and also the SiO₂ film 402 of the circuit area is removed during the cleaning operation. A gate insulating film 408 of the circuit area is oxidized again, and polycrystalline silicon is deposited as a gate electrode film 409 of the circuit area to form a resist pattern 410 corresponding to the gate electrode (FIG. 13C).

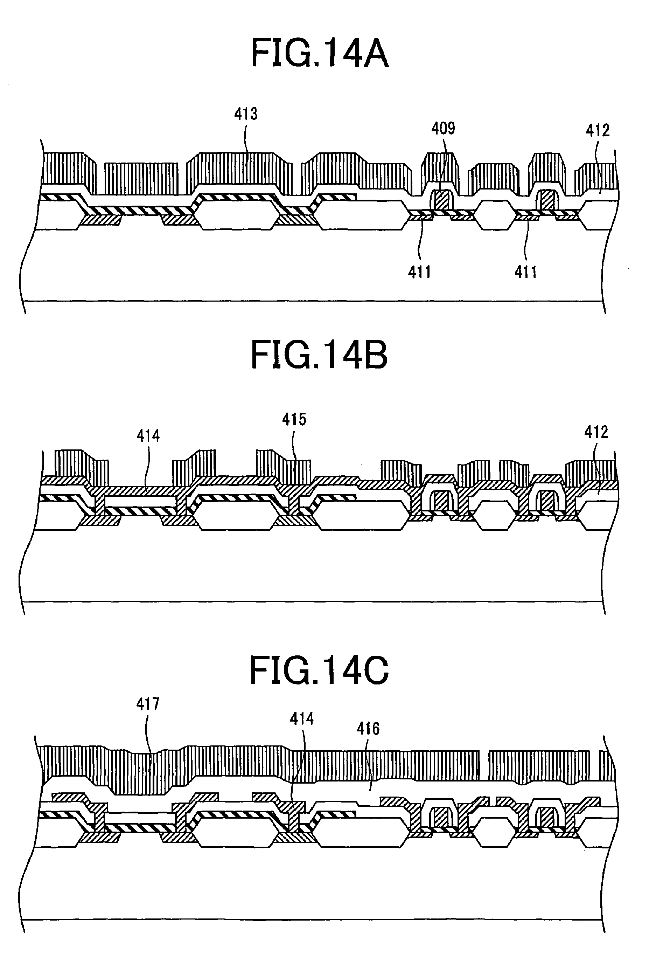

The gate electrode film 409 is formed by using the resist pattern 410 as a mask to form a source/drain diffusion layer 411 of the circuit area. Needless to say, the sensor area is protected at the time of ion implantation so as not to affect the concentration of a diffusion layer of the sensor area. Furthermore, a first inter-layer insulating film 412 is deposited, and a resist pattern 413 associated with opening portions for connecting the wiring to the diffusion layer both in the sensor area and the circuit area is formed (FIG. 14A). Contact holes are formed in the diffusion layers by using this resist pattern 413 as a mask, the resist is removed, cleaning is performed, and a metal film of a first wiring layer 414 is deposited to form a resist pattern 415. The first wiring layer 414 is dry-etched by using the resist pattern 415 as a mask, the resist is removed, cleaning is performed, and a second inter-layer insulating film 416 is deposited. Furthermore, a resist pattern 417 associated with a through hole for connecting a second wiring layer 418 to the first wiring layer 414 is formed (FIG. 14C).

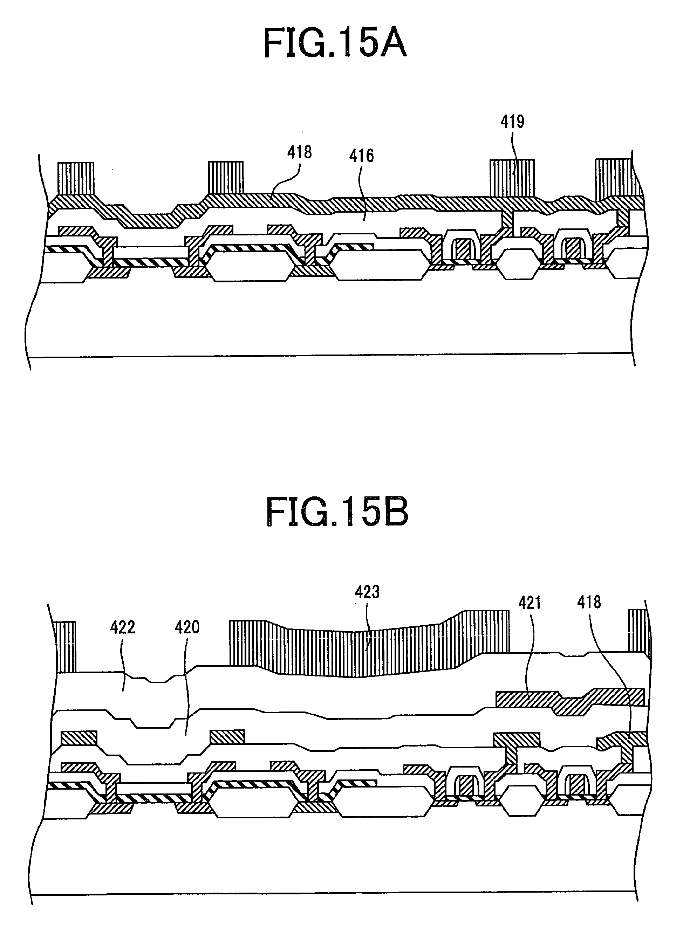

FIG. 15A illustrates the situation in which the through holes are formed in the second inter-layer insulating film 416, the resist pattern 417 is removed, cleaning is performed, and the second wiring layer 418 is deposited to form a resist pattern 419 associated with the wiring. The second wiring layer 418 is dry-etched, the resist is removed, cleaning is performed, and a third inter-layer insulating film 420 is deposited. Furthermore, the through-hole is worked and the wiring film is deposited and worked by the same method as that for forming the second wiring film to form the third wiring layer 421, and a passivation film 422 is deposited to form a resist pattern 423 for opening the Pad electrode of chip and the sensor area (FIG. 15B).

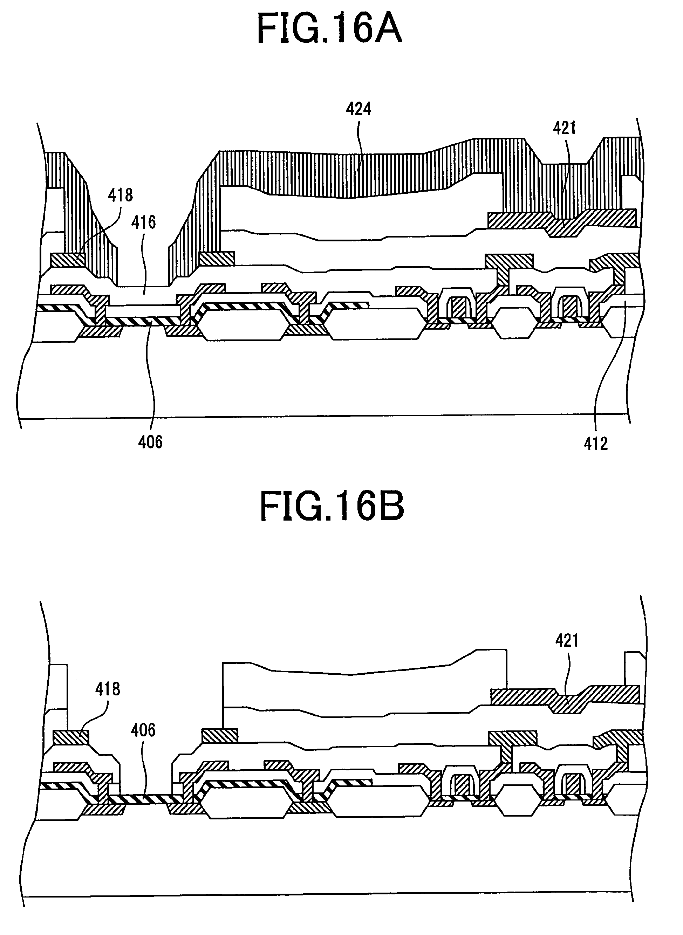

The passivation film 422 on the Pad electrode is worked in the circuit area and the passivation film 422 and the third inter-layer insulating film 420 are removed by etching in the sensor area. In this embodiment, the third inter-layer insulating film 420 is removed so as to connect a responsive electrode of the sensor to the second wiring layer 418. However, the configuration is allowable in which the responsive electrode is connected to an uppermost layer wiring (a third wiring layer 421) as described in the embodiment 1. FIG. 16A illustrates the situation in which a resist pattern 424 for exposing the gate insulating film of the sensor area is formed. The second inter-layer insulating film 416 and the first inter-layer insulating film 412 are etched and removed selectively with respect to the SRN film 406 by using the resist pattern 424 as a mask, the resist is removed, and cleaning is performed (FIG. 16B).

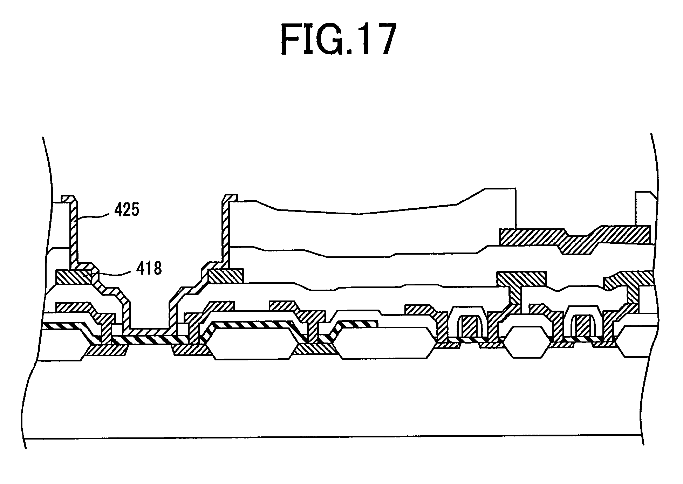

Then, a gas-responsive material electrode 425 is deposited using the stencil mask in such a way as to be connected to the second wiring layer 418 to manufacture a sensor (FIG. 17).

Reference numerals used in the figures of the present invention are described below.

101 ... Silicon substrate, 102 ... P-type well, 103 ... Isolation, 104 ... SiO₂, 105 ... Source/Drain diffusion layer, 106 ... P+ diffusion layer, 107 ... SRN film, 108 ... Inter-layer insulating film, 109 ... Resist pattern, 110 ... Resist pattern, 111 ... Second insulating film, 112 ... Al film, 113, 114 ... Resist pattern, 115 ... Electrode film, 116 ... Proton conductor, 300 ... Resist pattern, 305 ... Source/Drain diffusion layer, 306 ... P+ diffusion layer, 309 ... Resist pattern, 313 ... Resist pattern, 314 ... MOS transistor area, 315 ... Electrode film, 401 ... Silicon substrate, 402 ... SiO₂, 403 ... Isolation, 404 ... Source/Drain diffusion layer, 405 ... P+ diffusion layer, 406 ... SRN film, 407 ... Resist pattern, 408 ... Gate insulating film, 409 ... Gate electrode, 410 ... Resist pattern, 411 ... Source/Drain diffusion layer, 412 ... First inter-layer insulating film, 413 ... Resist pattern, 414 ... First wiring layer, 415 ... Resist pattern, 416 ... Second inter-layer insulating film, 417 ... Resist pattern, 418 ... Second wiring layer, 419 ... Resist pattern, 420 ... Third inter-layer insulating film, 421 ... Third wiring layer, 422 ... Passivation film, 423 ... Resist pattern, 424 ... Resist pattern, 425 ... Electrode of responsive material

## Claims

1. The semiconductor gas sensor comprising:
a semiconductor substrate (101; 401);
a gate insulating film (104; 402) formed on said semiconductor substrate (101; 401); and
a gate electrode (116; 402) having a material sensitive to a gas as an object for measurement formed on said gate insulating film (104; 402);
wherein said gate insulating film (104; 402) comprises a film stack of a silicon oxide film (104; 402) formed on said semiconductor substrate (101; 401) and a silicon nitride film (107; 406) provided on said silicon oxide film (104; 402), and
wherein said silicon nitride film (107; 406) is a silicon-rich nitride film (SRN) in which the composition ratio of silicon and nitrogen each constituting said silicon nitride film is equal to or greater than 1/2.

2. The semiconductor gas sensor according to Claim 1,
wherein said silicon nitride film (107; 406) is a silicon-rich nitride film (SRN) in which a composition ratio of silicon and nitrogen each constituting said silicon nitride film is greater than 3/4.

3. The semiconductor gas sensor according to Claim 1 or 2,
wherein said gate electrode (116; 402) comprises a metal material containing palladium or platinum.

4. The semiconductor gas sensor according to Claim 1 or 2,
wherein said gas as an object for measurement is hydrogen or methane gas.

5. The semiconductor gas sensor according to Claim 2, wherein the semiconductor gas sensor is formed of a MOS transistor (314) having a silicon dioxide gate insulating film (402) and a gate electrode of polycrystalline silicon formed on said semiconductor substrate (101; 401) with said semiconductor gas sensor formed thereon.

6. A method of producing the semiconductor gas sensor comprising steps of:
forming an isolation oxide film (103; 403) for separating each element electronically on the semiconductor substrate (101; 401);
selectively providing an area where a gas sensor is to be formed on said semiconductor substrate (101; 401) by using said isolation oxide film (103; 403);
forming a first gate insulating film (104; 402) on said area;
providing a pair of first diffusion zones (105, 106; 405, 406) at the position of holding said first gate insulating film (104; 402) in said area;
selectively providing an area where a circuit is to be formed on said semiconductor substrate (101; 401) after forming said first diffusion zone (105; 405);
forming a second gate insulating film (111) in said area where a circuit is to be formed on said semiconductor substrate; and
providing a pair of second diffusion areas (305, 306) having a conductive type opposite to a first conductive type at the position of holding said second gate insulating film (111) in said latter area;
wherein said first gate insulating film (104; 402) comprises a film stack of a silicon oxide film (104; 402) formed on said semiconductor substrate and a silicon nitride film (107; 406) provided on said silicon oxide film, and
wherein said silicon nitride film is a silicon-rich nitride film (SRN) in which the composition ratio of silicon and nitrogen each constituting said silicon nitride film is equal or greater than 1/2.

7. The method of producing the semiconductor gas sensor according to Claim 6,
wherein said silicon nitride film is a silicon-rich nitride film (SRN) in which composition ratio of silicon and nitrogene each constituting said silicon nitride film is greater than 3/4.

8. The method of producing the semiconductor gas sensor according to Claim 6 or 7 having steps:
forming a film stack of a dioxide silicon film and a silicon rich nitride as said first gate insulating film in said area where a circuit is to be formed;
forming an inter-layer insulating film (108; 412) so as to cover said film stack;
forming a wiring layer (414) on said inter-layer insulating film (108; 412);
selectively removing said inter-layer insulating film (108; 412) and exposing a surface of said film stack to form an opening portion; and
forming an electrode (425) made of a material sensitive to a gas as an object for measurement so as to cover the surface of the film stack of said opening portion and the side of said opening portion.
